# EUROPEAN PATENT SPECIFICATION

(11) **EP 0 998 906 B1**
(45) Date of publication and mention of the grant of the patent: **25.05.2005**
(21) Application number: 99120584.0
(22) Date of filing: 16.10.1999
(51) Int. Cl.: A61K 7/06, A61K 7/42

(54) **Shampoo composition containing light screening agents**
Lichtschutzmittel-enthaltende Shampoozusammensetzung
Composition de shampoing contenant des agents de protection de la lumière

(30) Priority: 26.10.1998 EP 98120226
(43) Date of publication of application: 10.05.2000
(73) Proprietor: DSM IP Assets B.V., 6411 TE Heerlen (NL)
(72) Inventor: Gonzenbach, Hans Ulrich, 1202 Geneva (CH); Krause, Martin, 01280 Prévessin-Moens (FR)
(74) Representative: Schwander, Kuno Josef, Dr.

(56) References cited:
- EP-A- 0 386 898
- EP-A- 0 842 965
- EP-A- 0 848 945
- EP-A- 0 920 859
- WO-A-98/13011
- US-A- 5 543 074

## Description

The invention relates to a shampoo composition containing a light screening agent which is resistant to being removed when the hair is washed.

More particularly, the invention relates to a shampoo composition containing a deposition enhancing agent and a light screening agent for the protection of the hair against ultraviolet rays of wavelengths between 280 and 320 nm (UV-B).

The European Patent Publication EP 0386 898 A1 teaches that enhanced levels of light screening agents can be deposited on hair surfaces using a shampoo which contains an anionic surfactant, a cationic derivative of a polygalactomannan gum as deposition enhancing agent and a water insoluble light screening agent of the p-methoxycinnamate type.

It has now been found that the substantivity of light screening agents in shampoo compositions is improved using a lipophilic polysiloxane type UV-B screening agent instead of a light screening agent of the p-methoxycinnamate type as described in the above cited European publication.

The term "substantivity" refers in the present context to resistance to removal from hair. Substantivity is the characteristic of a light screening agent that reflects how effectively the advertised degree of protection is maintained under repeated water exposure.

Thus, the present invention is concerned with a shampoo composition comprising 0.1 to 10.0 wt% of a cationic derivative of a polygalactomannan gum; and 0.1 to 10.0 wt% of a linear or cyclic polysiloxane compound of the general formula Ia or Ib, 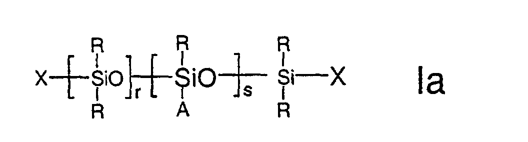 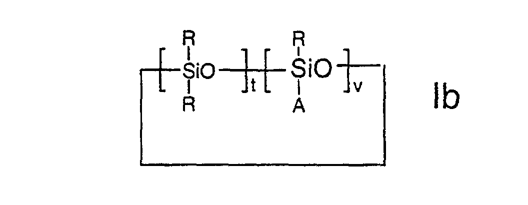 wherein
- X: signifies R or A;
- A: signifies a group of the formula IIa, IIb or IIc;

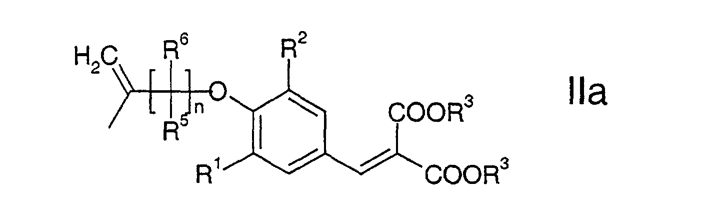

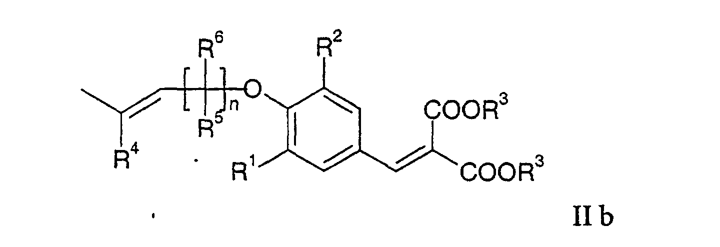

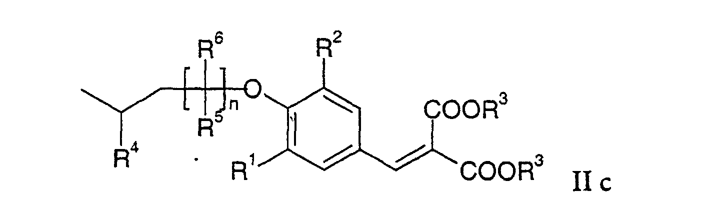

- R: signifies hydrogen, C₁₋₆ alkyl or phenyl;
- R¹ and R²: each independently signify hydrogen, hydroxy, C₁₋₆-alkyl or C₁₋₆-alkoxy;
- R³: signifies C₁₋₆-alkyl;
- R⁴: signifies hydrogen or C₁₋₆-alkyl;
- R⁵ and R⁶: each independently signify hydrogen or C₁₋₆-alkyl;
- r: has a value of from 0 to 250;
- s: has a value of from 0 to 20;
- r +s: has a value of at least 3;
- t: has a value of from 0 to 10;
- v: has a value of from 0 to 10; and
- v+t: has a value of at least 3;
- n: has a value from 1 to 6;
with the proviso that in the case that s is 0 at least one X is A and that s >0 X is R.

The term "C₁₋₆-alkyl" refers to groups such as methyl, ethyl, propyl, isopropyl, butyl, sec. butyl, isobutyl, pentyl and neopentyl. The term "C₁₋₆-alkoxy" refers to the corresponding alkoxy groups.

The residue R is preferably methyl.

The residues R¹ and R² are preferably hydrogen, methoxy or ethoxy, more preferably hydrogen, or one of R¹ and R² is hydrogen and the other is methoxy or ethoxy.

The residue R³ is preferably methyl or ethyl, more preferably ethyl.

Preferably, R⁴ is hydrogen or methyl, R⁵ and R⁶ are hydrogen and n is 1.

The polysiloxane compounds having a group A of the general formula IIa and IIb and their preparation are described in the European Patent EP 0538431 B1. These polysiloxane compounds are preferred.

The polysiloxane compounds having a group A of the general formula IIc and their preparation are described in the European Patent EP 0358584 B1.

In the linear polysiloxane compounds according to formula Ia the chromophore carrying residue A may be connected to the end groups of the polysiloxane (X=A) or may be statistically distributed (X=R).

Linear polysiloxane compounds wherein the chromophore carrying residue A is statistically distributed are preferred. Said preferred polysiloxane compounds have at least one unit carrying the chromophore residue (s=1), preferably s has a value of from 2 to 10, more preferably a statistical mean value of 4. The number of the other silicone units (r) present in the polysiloxane compounds is preferably 5 to 150, more preferably a statistical mean value of 60.

Polysiloxane compounds wherein 20% or less, preferably less than 10%, of the total siloxane units are units carrying a chromophore residue are preferred with respect to cosmetic properties.

The ratio of polysiloxane units having a chromophore residue A of the formula IIa to those units having a chromophore residue A of the formula IIb is 1:1 to 19:1, preferably 2:1 to 9:1, more preferably 4:1.

The concentration of the polysiloxane compound in the shampoo composition is preferably 0.1 to 10.0 wt%, more preferably 0.1 to 3.0 wt%.

Polygalactomannan gum, the principle component of the seed of the guar plant is a known stabilizer, thickening and film forming agent. The water soluble fraction of the guar plant extract consists of linear chains of (1→4)-β-D-mannopyranosyl units with α-D-galactopyranosyl units attached by (1→6)-linkage. The cationic derivatives of the polygalactomannan gum are obtained by reactions between the hydroxyl groups of the polygalactomannan and reactive quaternary ammonium compounds. An example of a suitable derivative is hydroxypropyltrimethylammonium guar commercially available from Henkel under the tradename COSMEDIA GUAR C261.

The concentration of the cationic derivative of the polygalactomannan gum is preferably 0.1 to 10.0 wt%, more preferably 0.1 to 5.0 wt%, most preferably 0.5 to 2 wt%.

The ratio of the cationic derivatives of the polygalactomannan gum to the polysiloxane compound as defined above is not critical. For example the ratio is 1:10 to 10:1, preferably 1:1.

The base ingredient of a shampoo composition is a water/anionic surfactant system that emulsifies the accumulated surface oils and removes them during the rinsing process. Thus, the shampoo composition further comprises an anionic surfactant.

Suitable anionic surfactants are C₁₀₋₁₈ -alkyl ether sulfates, C₁₀₋₁₈ -alkyl sulfates, sulfuric acid fatty alcohol esters and salts thereof like e.g. ammonium, sodium, potassium or mono-, di-or triethanolamine salts. Examples of C₁₀₋₁₈ -alkyl ether sulfates are sodium lauryl ether sulfate, potassium lauryl ether sulfate, ammonium lauryl ether sulfate. Examples of C₁₀₋₁₈ -alkyl sulfates are sodium lauryl sulfate (Sodium Laureth Sulfate), potassium lauryl sulfate and ammonium lauryl sulfate. Sodium Laureth Sulfate is available from Henkel under the tradenames TEXAPON N25 and TEXAPON N28.

Still further suitable anionic surfactants include α-olefin sulfonates, alkyl monoglyceride sulfonates, alkyl benzene sulfonates, alkyl sarcosinates, alkyl monoglyceride sulfates, monoalkylether sulfosuccinates, alkyl ether carboxylates and the like. The amount of anionic surfactant present in the shampoo influences the substantivity of the light screening agent. The anionic surfactant can be employed at concentrations within the range from 5 to 40 wt%, preferably 15 wt%.

The shampoo composition may also include other ingredients which are commonly employed in shampoos. Examples of such ingredients are:

Co-surfactants such as C₁₀₋₁₈ -alkyl or -alkylamido propylbetaine, C₁₀₋₁₈ -fatty acid alkanolamide or mixtures thereof. Examples of such co-surfactants are cocamidopropyl betaines available under the tradename TEGO-BETAIN L7 from Th. Goldschmidt or under the tradename DEHYTON K from Henkel;

Emulsifiers such as e.g. dialkanolamine alkyl phosphates (Amphisols) or polyoxyethylene derivatives e.g. Polysorbate 80, available from ICI under the tradename TWEEN 80. The emulsifier helps to increase the substantivity of above mentioned UV filter(s);

Cationic polymers such as e.g. Polyquaternium-7, available from Allied Colloids under the tradename SALCARE SC- 10. Cationic polymers also help to increase the substantivity of above mentioned UV filter(s);

Pearlizers such as e.g. EUPERLAN PK-3000 OK™ (a product containing Glycoldistearate, Glycerine and Laureth-4) available from Henkel;

Refatting agents such as e.g. PEG-7 Glyceryl Cocoate available under the tradename CETIOL HE from Henkel;

Viscosity adjusting agents such as e.g. Laureth-3 available from Hoechst under the tradename GENAPOL L-3 or Laureth-2 available from Henkel under the tradename DEHYDOL LS-2.

The commonly employed ingredients listed above may be added in an amount of 0.1 to 20.0 wt%.

Examples of further ingredients commonly employed in shampoos are: foam boosters, opacifiers, fragance, dyes, coloring agents, conditioning agents, preservatives, proteins, buffering agents.

In addition to the polysiloxane compound of the general formula Ia or Ib, the shampoo composition may contain other known UV-A and/or UV-B filters in an amount of 0.1 to 10.0 wt%.

Suitable UV B filters, i.e. substances having absorption maxima between 290 and 320 nm, are for example the following organic compounds:
--- p-Aminobenzoic acid derivatives such as ethyl, propyl, butyl and isobutyl p-aminobenzoate;
--- Acrylates such as 2-ethylhexyl 2-cyano-3,3-diphenylacrylate (octocrylene), ethyl 2-cyano-3,3-diphenylacrylate;
--- Aniline derivatives such as methyl anilinum methosulfate;
--- Anthranilic acid derivatives such as menthyl anthranilate;
--- Benzophenone derivatives such as benzophenone-3, benzophenone-4;
--- Camphor derivatives such as 4-methyl benzylidene camphor (PARSOL 5000™), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid;
--- Cinnamate derivatives such as octyl methoxycinnamate (PARSOL MCX™), ethoxyethyl methoxycinnamate, diethanolamine methoxycinnamate (PARSOL Hydro™), isoamyl methoxycinnamate and the like as well as cinnamic acid derivatives bond to siloxanes;
--- Gallic acid such as digalloyl trioleate;
--- Imidazole derivatives such as 2-phenyl benzimidazole sulfonic acid and its salts (PARSOL HS™). Salts of 2-phenyl benzimidazole sulfonic acid are e.g. alkali salts such as sodium- or potassium salts, ammonium salts, morpholine salts, salts of primary, sec. and tert. amines like monoethanolamine salts, diethanolamine salts;
--- Salicylate derivatives such as isopropylbenzyl salicylate, benzyl salicylate, butyl salicylate, octyl salicylate (NEO HELIOPAN OS™), isooctyl salicylate, homomenthyl salicylate (homosalate, HELIOPAN™);
--- Triazole derivatives such as hydroxydibutylphenyl benztriazole (KEMISORB 72™);
--- Triazone derivatives such as octyl triazone (UVINUL T-150™), dioctyl butamido triazone (UVASORB HEB™);
--- Organosiloxane compounds containing benzmalonate groups as described in the European Patent Publications EP 0358584 B1, EP 0538431 B1 and EP 0709080 A1; and
--- Pigments such as e.g. microparticulated TiO_{2.} The term "microparticulated" refers to a particle size from 5 nm to 200 nm, particularly from 15 nm to 100 nm. The TiO₂ particles may also be coated by metal oxides such as e.g. aluminum or zirconium oxides or by organic coatings such as e.g. polyols, methicone, aluminum stearate, alkyl silane. Such coatings are well known in the art.

The composition may further contain UV-A filters such as
--- Dibenzoylmethane derivatives such as 4-tert. butyl-4-methoxydibenzoylmethane (PARSOL 1789™), dimethoxydibenzoylmethane, isopropyldibenzoylmethane;
--- Triazine compounds as described in the European Patent Publications EP 0693 483 A1, EP 0704 437 A2, EP 0704 444 A1 and EP 0780 382 A1;
--- Benzotriazole derivatives such as 2,2'-methylene-bis-(6-(2H-benzotriazole-2-yl)-4-(1,1,3,3,-tetramethylbutyl)-phenol (TINOSORB M™); and
--- Pigments such as e.g. microparticulated ZnO. The term "microparticulated" refers to a particle size from 5 nm to 200 nm, particularly from 15 nm to 100 nm. The ZnO particles may also be coated by metal oxides such as e.g. aluminum or zirconium oxides or by organic coatings such as e.g. polyols, methicone, aluminum stearate, alkyl silane. Such coatings are well known in the art.

A suitable shampoo composition of the invention contains e.g. the following ingredients at levels of % active ingredient:

| | |
|---|---|
| Anionic surfactant | ∼ 11.0 |
| Amphoteric surfactant | ∼ 3.0 |
| Pearlizer | ∼ 2.0 |
| Thickener | ∼ 0 - 3.0 |
| UV filter | ∼ 0.25 - 1.0 |
| Deposition enhancer | ∼ 0.1 -3.0 |
| Water demin. | q.s. |

A preferred shampoo composition comprises
0.1 to 10.0 wt% of a cationic derivative of a polygalactomannan gum; and
0.1 to 10.0 wt% of a linear polysiloxane compound of the general formula Ia,
wherein
- X: signifies methyl;
- A: signifies a group of the formula IIa or IIb;
- R: signifies methyl;
- R¹ and R²: signify hydrogen, methoxy or ethoxy, or one of R¹ and R² is hydrogen and the other is methoxy or ethoxy;
- R³: signifies methyl or ethyl;
- R⁴: signifies hydrogen or methyl;
- R⁵ and R⁶: signify hydrogen;
- r: is 5 to 150;
- s: is 2 to 10;
- n: has a value of 1.

A more preferred shampoo composition contains
0.1 to 10 wt% of a cationic derivative of a polygalactomannan gum; and
0.1 to 10 wt% of a linear polysiloxane compound of the general formula Ia,
wherein
- X: signifies methyl;
- A: signifies a group of the formula IIa or IIb;
- R: signifies methyl;
- R¹ and R²: signify hydrogen;
- R³: signifies ethyl;
- R⁴: signifies hydrogen;
- R⁵ and R⁶: signify hydrogen;
- s: is a statistical mean value of 4;
- r: is a statistical mean value of 60;
- n: has a value of 1.

The polysiloxane compounds Ia or Ib wherein A is a residue of the formula IIa or IIb can be prepared as described in EP 0538431 B 1 by silylation of the corresponding benzalmalonates according to the following reaction scheme: 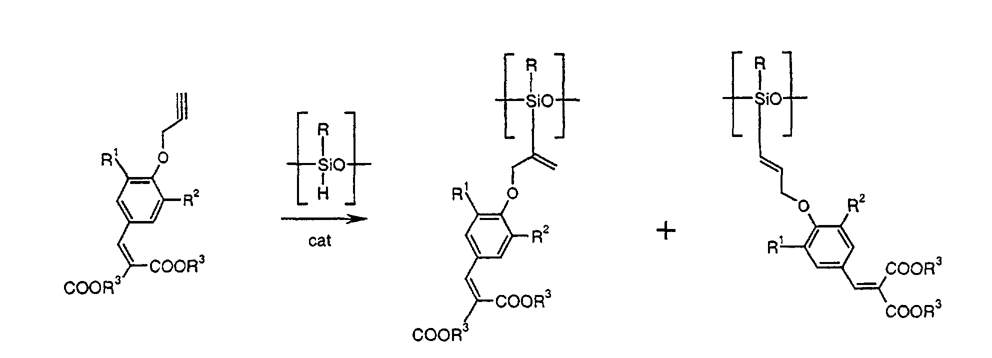 wherein R, R¹, R², and R³ are as defined above.

The reaction between a polysiloxane wherein A signifies hydrogen and the 4-(2-propynylox)phenyl methylene diethylester may be carried out employing known procedures for the addition of silicon bonded hydrogen atoms to groups containing aliphatic unsaturation. Such reactions are generally catalyzed by a platinum group metal or a complex of such a metal. Examples of catalysts which may be employed are platinum on carbon, chloroplatinic acid, platinum acetyl acetonate, complexes of platinum compounds with unsaturated compounds e.g. olefins and divinyl disiloxanes, complexes of rhodium and palladium compounds and complexes of platinum compounds supported on inorganic substrates. The addition reaction may be performed at reduced, atmospheric or increased pressure. A solvent can be used, e.g. toluene or xylene, in the reaction mixture although the presence of the solvent is not essential. It is also preferred to carry out the reaction at elevated reaction temperatures e.g. from 50°C up to 150°C.

The production of the novel shampoo composition maybe effected in a manner known per se according to the so called "cold process" or "hot process".

In the cold process the surfactants are dissolved in deionized water under stirring. Then the cationic derivative of the polygalactomannan gum, the polysiloxane compound of the general formula Ia or Ib and other ingredients as listed above are added. The mixture is stirred until a uniform solution is obtained. The pH is adjusted to 6-7. Salts such as e.g. sodium chloride or other viscosity modifiers are added to adjust the flow characteristics. Optionally fragance and preservatives are added.

The hot process comprises the following steps:
A: The surfactant compounds are dissolved in deionized water under stirring and optionally under heating.
B: The oil-soluble components (cationic derivative of the polygalactomannan gum, the polysiloxane compound of the general formula Ia or Ib and other ingredients as listed above) are heated to a temperature of approximately 10° C above the melting point of the components. The oil soluble components may be added together or separately. Phase B is added to Phase A under stirring. The composition is then cooled. The pH is adjusted to 6-7. Optionally fragance and preservatives are added.
C: Salt or viscosity modifiers are added to adjust the flow characteristics.

Better stability is generally found with the hot process, due to the finer dispersion of particles that is achieved through the emulsification process.

The wash off resistance test measures the ability of the light screening agent to resist being washed off the hair by a solution of liquid detergent. The test is effected as follows: Locks of hair are washed with the UV filter containing shampoo, then dried and immersed in a glass recipient in e.g. tetrahydrofuran (THF), the recipient is put into an ultrasonic bath for one hour to extract the light screening agent from the hair. The THF is removed, the locks of hair are rinsed with THF. The light screening content is measured in the combined extracts using HPLC Chromatography. The substantivity is given in µg light screening agent pro 1 g hair.

The following tables explain the invention in more detail:

In the shampoo compositions listed in the tables below the following UV filters have been selected
PARSOL SLX™: a compound of the general formula Ia, wherein
   - X: signifies methyl;
   - A: signifies a group of the formula IIa or IIb;
   - R: signifies methyl;
   - R¹ and R²: signify hydrogen;
   - R³: signifies ethyl;
   - R⁴: signifies hydrogen;
   - R⁵ and R⁶: signify hydrogen;
   - s: is a statistical mean value of 4;
   - r: is a statistical mean value of 60;
   - n: has a value of 1.
PARSOL MCX™: 2-ethylhexyl-p-methoxycinnamate.

**Table 1**

| Ingredient | Ex. 1 | Ex. 2 |
|---|---|---|
| | wt% | wt% |
| TEXAPON N28™ | 35.00 | 35.00 |
| CETIOL HE™ | 4.00 | 4.00 |
| PARSOL SLX™ | 1.00 | |
| PARSOL MCX™ | | 1.00 |
| COSMEDIA GUAR C261™ | 0.50 | 0.50 |
| Deionized Water | 51.5 | 51.5 |
| TEGO-BETAIN L7™ | 8.00 | 8.00 |
| NaCl | up to 100 | up to 100 |
| | | |
| Substantivity µg/g | 1299.1 | 437.8 |

Table 1 shows the improved substantivity of PARSOL SLX™ in a shampoo composition prepared according to the cold process (Example 1) compared to the substantivity of PARSOL MCX™ in the corresponding shampoo composition (Example 2).

**Table 2**

| Process step | Ingredient | Ex. 3 | Ex. 4 | Ex. 5 | Ex. 6 | Ex. 7 | Ex. 8 |
|---|---|---|---|---|---|---|---|
| | | wt% | wt% | wt% | wt% | wt% | wt% |
| A | TEXAPON N28™ | 35.00 | 35.00 | 35.00 | 35.00 | 35.00 | 35.00 |
| | Deionized Water | 50.45 | 50.95 | 51.20 | 46.90 | 49.22 | 49.89 |
| | | | | | | | |
| B | CETIOL HE™ | 4.00 | 4.00 | 4.00 | 4.00 | 4.00 | 4.00 |
| B | PARSOL SLX™ | 1.00 | 0.50 | 0.25 | 1.00 | 1.00 | 1.00 |
| B | COSMEDIA GUAR C261™ | 0.55 | 0.55 | 0.55 | 1.10 | 0.28 | 0.11 |
| B | TEGO-BETAIN L7™ | 8.00 | 8.00 | 8.00 | 8.00 | 8.00 | 8.00 |
| C | GENAPOL L-3™ | 1.00 | 1.00 | 1.00 | - | 1.50 | 2.00 |
| | | 100.00 | 100.00 | 100.00 | 100.00 | 100.00 | 100.00 |
| | | | | | | | |
| | Substantivity µg/g | 850.9 | 408.9 | 283.6 | 1237.0 | 593.7 | 189.1 |

Table 2 shows the substantivity of PARSOL SLX™ in a shampoo formulation prepared according to the hot process using different concentrations of PARSOL SLX™ and of COSMEDIA GUAR C261™ (Examples 3-8).

**Table 3**

| Process step | | Ex.9 | Ex.10 | Ex.11 | Ex.12 |
|---|---|---|---|---|---|
| | | wt% | wt% | wt% | wt% |
| A | TEXAPON N28™ | 25.00 | 15.00 | 35.00 | 35.00 |
| | Deionized Water | 59.45 | 69.45 | 47.45 | 52.45 |
| B | CETIOL HE™ | 4.00 | 4.00 | 4.00 | 4.00 |
| B | PARSOL SLX™ | 1.00 | 1.00 | 1.00 | 1.00 |
| B | COSMEDIA GUAR C261™ | 0.55 | 0.55 | 0.55 | 0.55 |
| B | TEGO-BETAIN L7™ | 8.00 | 8.00 | 15.00 | 4.00 |
| C | GENAPOL L-3™ | 2.00 | 2.00 | 3.00 | 3.00 |
| | | | | | |
| | | 100.00 | 100.00 | 100.00 | 100.00 |
| | | | | | |
| | Substantivity µg/g | 499.0 | 708.2 | 593.5 | 596.1 |

Table 3 shows the substantivity of PARSOL SLX™ in a shampoo formulation prepared according to the hot process using different concentrations of surfactant TEXAPON N28™ and TEGO-BETAIN L7™ (Examples 9 -12).

**Table 4**

| Ingredient | Ex. 13 | Ex. 14 | Ex. 15 | Ex. 16 |
|---|---|---|---|---|
| | wt% | wt% | wt% | wt% |
| TEXAPON N25™ | 30.00 | 30.00 | 30.00 | 30.00 |
| CETIOL HE™ | 3.00 | 3.00 | 3.00 | 3.00 |
| PARSOL SLX™ | 0.5 | 0.5 | 1 | 1 |
| COSMEDIA GUAR C261™ | 0.11 | 0.11 | 0.11 | 0.11 |
| SALCARE SC-10™ | 1.50 | 1.50 | 1.50 | 1.50 |
| AMPHISOL NP™ | 0.50 | 0.50 | 0.50 | 0.50 |
| EUPERLAN 3000 OK™ | 3.00 | 3.00 | 3.00 | 3.00 |
| TWEEN80™ | 1.00 | 1.00 | 1.00 | 1.00 |
| Deionized water | 51.39 | 51.39 | 50.89 | 50.89 |
| DEHYTON K™ | 8.00 | 8.00 | 8.00 | 8.00 |
| DEHYDOL LS 2™ | 1.00 | 1.00 | 1.00 | 1.00 |
| | | | | |
| Process | Hot (75°C) | cold | hot (75°C) | cold |
| Substantivity µg/g | 360.10 | 774.20 | 729.80 | 1139.30 |

Table 4 shows the substantivity of PARSOL SLX™ in a shampoo formulation prepared according to the hot process (Example 13 and 15) and according to the cold process (Examples 14 and 16).

The substantivity test was effected as follows:
Blond Caucasian, virgin untreated hair obtained from Fischer & Miller Laupheim, Germany was washed with the UV filter containing shampoo. Each hair switch was then dried and introduced into a brown glass recipient of 60 ml. After adding 30 ml of tetrahydrofuran (THF), the recipient was put into an ultrasonic bath for one hour to extract the UV filter. THF was then evaporated and the residues analyzed by HPLC chromatography for its UV filter content.

## Claims

1. Shampoo composition comprising 0.1 to 10.0 wt% of a cationic derivative of a polygalactomannan gum; and 0.1 to 10.0 wt% of a linear or cyclic polysiloxane compound of the general formula Ia or Ib, 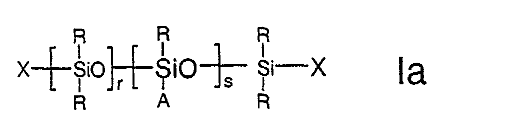 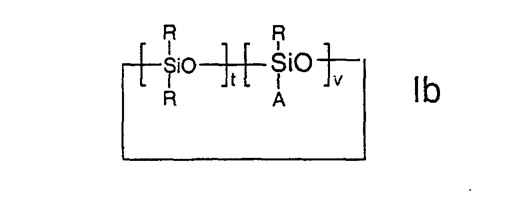 wherein
X signifies R or A;
A signifies a group of the formula IIa, IIb or IIc; 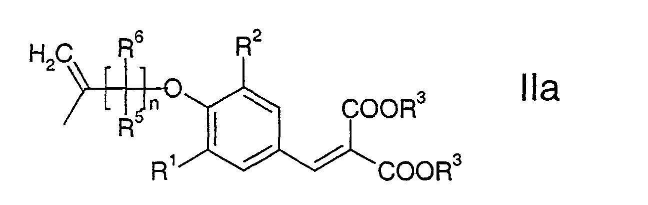 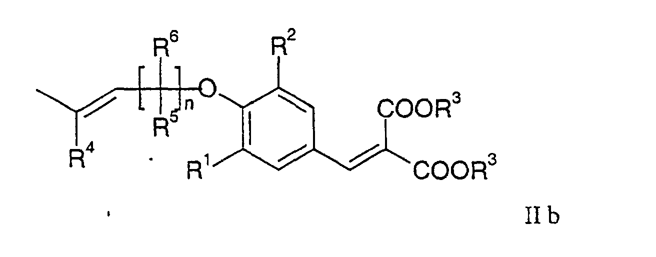 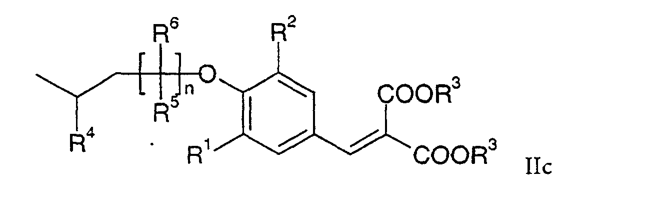
R signifies hydrogen, C₁₋₆ alkyl or phenyl;
R¹ and R² each independently signify hydrogen, hydroxy, C₁₋₆-alkyl or C₁₋₆-alkoxy;
R³ signifies C₁₋₆-alkyl;
R⁴ signifies hydrogen or C₁₋₆-alkyl;
R⁵ and R⁶ each independently signify hydrogen or C₁₋₆-alkyl;
r has a value of from 0 to 250;
s has a value of from 0 to 20;
r +s has a value of at least 3;
t has a value of from 0 to10;
v has a value of from 0 to 10; and
v+t has a value of at least 3;
n has a value from 1 to 6;
with the proviso that in the case that s is 0 at least one X is A and that s >0 X is R.

2. Shampoo composition according to claim 1, wherein A signifies a group of the formula IIa or IIb.

3. Shampoo composition according to claim 2, wherein the ratio of polysiloxane units having a chromophore residue A of the formula IIa to those units having a chromophore residue A of the formula IIb is 1:1 to 19:1, preferably 2:1 to 9:1, more preferably 4:1.

4. Shampoo composition according to any one of claims 1 to 3, wherein R signifies methyl.

5. Shampoo composition according to any one of claims 1 to 4, wherein R¹ and R² each independently signify hydrogen, methoxy or ethoxy.

6. Shampoo composition according to claim 5, wherein R¹ and R² signify hydrogen.

7. Shampoo composition according to claim 5, wherein one of R¹ and R² signifies hydrogen and the other signifies methoxy or ethoxy.

8. Shampoo composition according to any one of claims 1 to 7, wherein R³ signifies methyl or ethyl.

9. Shampoo composition according to any one of claims 1 to 8, wherein R⁴ signifies hydrogen or methyl.

10. Shampoo composition according to any one of claims 1 to 9, wherein R⁵ and R⁶ signify hydrogen

11. Shampoo composition according to any one of claims 1 to 10, wherein n is 1.

12. Shampoo composition according to any one of claims 1-11, wherein the concentration of the polysiloxane compound is preferably 0.1 to 3.0 wt% and the concentration of the cationic derivative of the polygalactomannan gum is 0.1 to 5.0 wt%, preferably 0.5 to 2 wt%.

13. Shampoo composition according to any one of claims 1-12, wherein the polysiloxane compound has the general formula Ia and
A signifies a group of the formula IIa or IIb
X signifies methyl;
R signifies methyl;
R¹ and R² signify hydrogen, methoxy or ethoxy, or one of R¹ and R² is hydrogen and the other is methoxy or ethoxy;
R³ signifies methyl or ethyl;
R⁴ signifies hydrogen or methyl;
R⁵ and R⁶ signify hydrogen;
r is 5 to 150;
s is 2 to 10;
n has a value of 1.

14. Shampoo composition according to claim 13, wherein
R¹ and R² signify hydrogen;
R³ signifies ethyl;
R⁴ signifies hydrogen;
s is a statistical mean value of 4;
r is a statistical mean value of 60.

15. Shampoo composition according to any one of claims 1-14, wherein said composition contains in addition common UV-A filter and/or UV-B filter.

## Patentansprüche

1. Shampoo-Zusammensetzung, umfassend 0,1 bis 10,0 Gewichtsprozent eines kationischen Derivats von einem Polygalactomannangummi, und 0,1 bis 10,0 Gewichtsprozent einer linearen oder cyclischen Polysiloxanverbindung der allgemeinen Formel Ia oder Ib 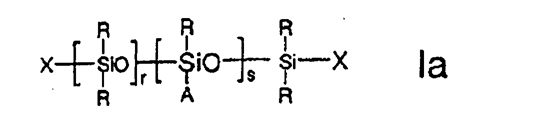 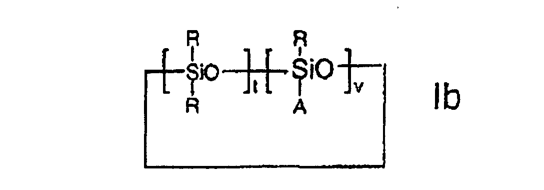 worin
X R oder A bedeutet;
A eine Gruppe der Formel IIa, IIb oder IIc 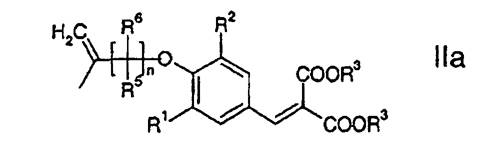 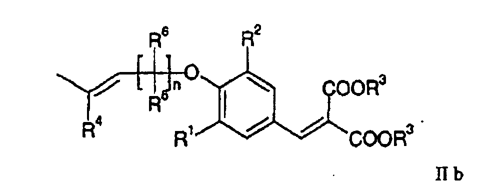 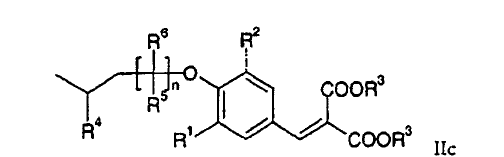
bedeutet;
R Wasserstoff, C₁₋₆-Alkyl oder Phenyl bedeutet;
R¹ und R² jeweils unabhängig Wasserstoff, Hydroxy, C₁₋₆-Alkyl oder C₁₋₆-Alkoxy bedeuten;
R³ C₁₋₆-Alkyl bedeutet;
R⁴ Wasserstoff oder C₁₋₆-Alkyl bedeutet;
R⁵ und R⁶ jeweils unabhängig Wasserstoff oder C₁₋₆-Alkyl bedeuten;
r einen Wert von 0 bis 250 aufweist;
s einen Wert von 0 bis 20 aufweist;
r+s einen Wert von mindestens 3 aufweist;
t einen Wert von 0 bis 10 aufweist;
v einen Wert von 0 bis 10 aufweist; und
v+t einen Wert von mindestens 3 aufweist;
n einen Wert von 1 bis 6 aufweist;
mit der Maßgabe, dass in dem Fall, dass s 0 ist, mindestens einer von X A ist, und in dem Fall, dass s >0, X R darstellt.

2. Shampoo-Zusammensetzung nach Anspruch 1, worin A eine Gruppe der Formel IIa oder IIb wiedergibt.

3. Shampoo-Zusammensetzung nach Anspruch 2, worin das Verhältnis von Polysiloxaneinheiten mit einem Chromophorenrest A der Formel IIa zu jenen Einheiten mit einem Chromophorenrest A der Formel IIb 1:1 bis 19:1, vorzugsweise 2:1 bis 9:1, bevorzugter 4:1, ist.

4. Shampoo-Zusammensetzung nach einem der Ansprüche 1 bis 3, worin R Methyl bedeutet.

5. Shampoo-Zusammensetzung nach einem der Ansprüche 1 bis 4, worin R¹ und R² jeweils unabhängig Wasserstoff, Methoxy oder Ethoxy bedeuten.

6. Shampoo-Zusammensetzung nach Anspruch 5, worin R¹ und R² Wasserstoff bedeuten.

7. Shampoo-Zusammensetzung nach Anspruch 5, worin einer von R¹ und R² Wasserstoff bedeutet und der andere Methoxy oder Ethoxy bedeutet.

8. Shampoo-Zusammensetzung nach einem der Ansprüche 1 bis 7, worin R³ Methyl oder Ethyl bedeutet.

9. Shampoo-Zusammensetzung nach einem der Ansprüche 1 bis 8, worin R⁴ Wasserstoff oder Methyl bedeutet.

10. Shampoo-Zusammensetzung nach einem der Ansprüche 1 bis 9, worin R⁵ und R⁶ Wasserstoff bedeuten.

11. Shampoo-Zusammensetzung nach einem der Ansprüche 1 bis 10, worin n 1 ist.

12. Shampoo-Zusammensetzung nach einem der Ansprüche 1 bis 11, worin die Konzentration der Polysiloxanverbindung vorzugsweise 0,1 bis 3,0 Gewichtsprozent ist und die Konzentration des kationischen Derivats von dem Polygalactomannangummi 0,1 bis 5,0 Gewichtsprozent, vorzugsweise 0,5 bis 2 Gewichtsprozent, ist.

13. Shampoo-Zusammensetzung nach einem der Ansprüche 1 bis 12, worin die Polysiloxanverbindung die allgemeine Formel Ia aufweist und
A eine Gruppe der Formel IIa oder IIb bedeutet;
X Methyl bedeutet;
R Methyl bedeutet;
R¹ und R² Wasserstoff, Methoxy oder Ethoxy bedeuten, oder einer von R¹ und R² Wasserstoff bedeutet und der andere Methoxy oder Ethoxy bedeutet;
R³ Methyl oder Ethyl bedeutet;
R⁴ Wasserstoff oder Methyl bedeutet;
R⁵ und R⁶ Wasserstoff bedeuten;
r 5 bis 150 ist;
s 2 bis 10 ist;
n einen Wert von 1 aufweist.

14. Shampoo-Zusammensetzung nach Anspruch 13, worin
R¹ und R² Wasserstoff bedeuten;
R³ Ethyl bedeutet;
R⁴ Wasserstoff bedeutet;
s ein statistischer Mittelwert von 4 ist;
r ein statistischer Mittelwert von 60 ist.

15. Shampoo-Zusammensetzung nach einem der Ansprüche 1 bis 14, worin die Zusammensetzung zusätzlich üblichen UV-A-Filter und/oder UV-B-Filter enthält.

## Revendications

1. Composition pour shampoing comprenant de 0,1 à 10,0 % en poids d'un dérivé cationique d'un polygalactomannane (gomme) ; et de 0,1 à 10,0 % en poids d'un composé de polysiloxane linéaire ou cyclique de formule générale Ia ou Ib, 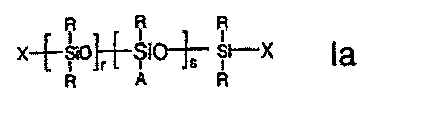 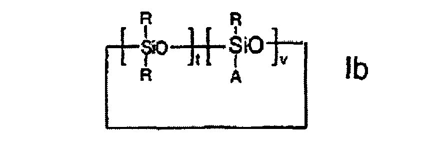 où
X est R ou A ;
A est un groupe de formule IIa, IIb ou IIc ; 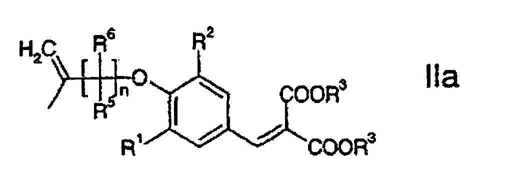 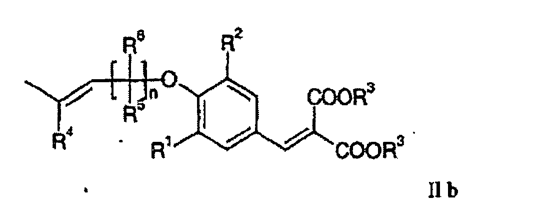 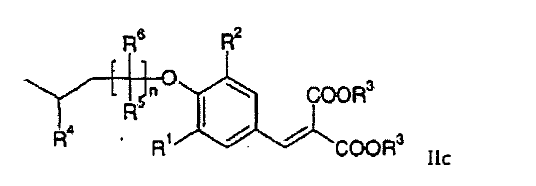
R est un hydrogène, un phényle ou un alkyle en C₁₋₆ ;
R¹ et R² sont chacun indépendamment un hydrogène, un hydroxy, un alkyle en C₁₋₆ ou un alcoxy en C₁₋₆ ;
R³ est un alkyle en C₁₋₆ ;
R⁴ est un hydrogène ou un alkyle en C₁₋₆ ;
R⁵ et R⁶ sont chacun indépendamment un hydrogène ou un alkyle en C₁₋₆ ;
r prend une valeur comprise entre 0 et 250 ;
s prend une valeur comprise entre 0 et 20 ;
r+s prend une valeur au moins égale à 3 ;
t prend une valeur comprise entre 0 et 10 ;
v prend une valeur comprise entre 0 et 10 ; et
v+t prend une valeur au moins égale à 3 ;
n prend une valeur comprise entre 1 et 6
à condition que dans le cas où s équivaut à 0, au moins un X est A, et que lorsque s > 0, X est R.

2. Composition pour shampoing selon la revendication 1, dans laquelle A est un groupe de formule IIa ou IIb.

3. Composition pour shampoing selon la revendication 2, dans laquelle le rapport des unités de polysiloxane comportant un résidu chromophore A de formule IIa sur les unités comportant un résidu chromophore A de formule IIb est de 1:1 à 19:1, de préférence de 2:1 à 9:1, de préférence de 4:1.

4. Composition pour shampoing selon l'une quelconque des revendications 1 à 3, dans laquelle R est un méthyle.

5. Composition pour shampoing selon l'une quelconque des revendications 1 à 4, dans laquelle R¹ et R² sont chacun indépendamment un hydrogène, un méthoxy ou un éthoxy.

6. Composition pour shampoing selon la revendication 5, dans laquelle R¹ et R² sont un hydrogène.

7. Composition pour shampoing selon la revendication 5, dans laquelle l'un des deux radicaux R¹ ou R² est un hydrogène et l'autre est un méthoxy ou un éthoxy.

8. Composition pour shampoing selon l'une quelconque des revendications 1 à 7, dans laquelle R³ est un méthyle ou un éthyle.

9. Composition pour shampoing selon l'une quelconque des revendications 1 à 8, dans laquelle R⁴ est un hydrogène ou un méthyle.

10. Composition pour shampoing selon l'une quelconque des revendications 1 à 9, dans laquelle R⁵ et R⁶ sont un hydrogène.

11. Composition pour shampoing selon l'une quelconque des revendications 1 à 10, dans laquelle n équivaut à 1.

12. Composition pour shampoing selon l'une quelconque des revendications 1 à 11, dans laquelle la concentration du composé de polysiloxane s'élève de préférence de 0,1 à 3,0 % en poids et la concentration du dérivé cationique du polygalactomannane (gomme) de 0,1 à 5,0 % en poids, de préférence de 0,5 à 2 % en poids.

13. Composition pour shampoing selon l'une quelconque des revendications 1 à 12, dans laquelle le composé de polysiloxane est de formule générale Ia et
A est un groupe de formule IIa ou IIb ;
X est un méthyle ;
R est un méthyle ;
R¹ et R² sont chacun un hydrogène, un méthoxy ou un éthoxy, ou l'un des deux radicaux R¹ ou R² est un hydrogène et l'autre est un méthoxy ou un éthoxy ;
R³ est un méthyle ou un éthyle ;
R⁴ est un hydrogène ou un méthyle ;
R⁵ et R⁶ sont un hydrogène ;
r prend une valeur comprise entre 5 et 150 ;
s prend une valeur comprise entre 2 et 10 ;
n équivaut à 1.

14. Composition pour shampoing selon la revendication 13, dans laquelle
R¹ et R² sont de l'hydrogène ;
R³ est de l'éthyle ;
R³ est de l'hydrogène ;
s est une valeur moyenne statistique équivalant à 4 ;
r est une valeur moyenne statistique équivalant à 60.

15. Composition pour shampoing selon l'une quelconque des revendications 1 à 14, dans laquelle ladite composition contient en outre un filtre UV-A et/ou un filtre UV-B commun.
